**Europäisches Patentamt**

**European Patent Office**

**Office européen des brevets**

(19)

(11) Veröffentlichungsnummer : **0 044 574**
**B1**

# EUROPÄISCHE PATENTSCHRIFT

(12)

(45) Veröffentlichungstag der Patentschrift :
**15.08.84**

(21) Anmeldenummer : **81106920.2**

(22) Anmeldetag : **16.01.80**

(60) Veröffentlichungsnummer der früheren Anmeldung
nach Art. 76 EPÜ : **0014343**

(51) Int. Cl.³ : **B 01 J 20/10**, C 01 B 33/24,
B 01 D 53/02

(54) **Verwendung von Calciumsilikatgranulaten bzw. -pulvern zur hydrophilen Ab- bzw. Adsorption.**

(30) Priorität : **19.01.79 DE 2902108**

(43) Veröffentlichungstag der Anmeldung :
**27.01.82 Patentblatt 82/04**

(45) Bekanntmachung des Hinweises auf die Patenterteilung : **15.08.84 Patentblatt 84/33**

(84) Benannte Vertragsstaaten :
**AT BE CH FR GB IT LI LU NL SE**

(56) Entgegenhaltungen :
**CH-A- 359 690**
**DE-A- 2 640 341**
**GB-A- 1 301 172**
**US-A- 2 966 441**
**US-A- 3 144 346**
**CHEMICAL ABSTRACTS, Band 82, 1975, Seite 338,
Nr. 102885c Columbus, Ohio, U.S.A.**
**Die Akte enthält technische Angaben, die nach dem
Eingang der Anmeldung eingereicht wurden und die
nicht in dieser Patentschrift enthalten sind.**

(73) Patentinhaber : **MARS INCORPORATED
Westgate Park 1651 Old Meadow Road
McLean Virginia 22102 (US)**

(72) Erfinder : **Krämer, Walter
Uhlenkamp 1
D-3040 Soltau-Friedrichseck (DE)**
Erfinder : **Follmann, Rainer, Dr. Dipl.-Chem.
Blumenstrasse 22
D-4950 Minden (DE)**

(74) Vertreter : **Hoormann, Walter, Dr. et al
FORRESTER & BOEHMERT Widenmayerstrasse 4/I
D-8000 München 22 (DE)**

**Beschreibung**

Die Erfindung betrifft die Verwendung von weiter unten festgelegten Calciumsilikatgranulaten bzw. -pulvern zur hydrophilen Ab- bzw. Adsorption.

Bei den für den Verwendungszweck der Flüssigkeitsabsorption bzw. -adsorption eingesetzten bekannten Produkten handelt es sich im wesentlichen um Naturprodukte mineralischen oder organischen Ursprunges, wie Bimsstein, Tonmineralien, beispielsweise Sepiolithe, Holzmehle und Kieselgur, die aufgrund ihrer natürlichen Porosität hydrophile und/oder lipophile Eigenschaften besitzen. Organische Produkte haben jedoch den Nachteil von unerwünschten Nebenwirkungen bei ihrer Verwendung, wie ihre Brennbarkeit. Andererseits neigen mineralische Produkte, wie Tonmineralien, durch Aufnahme von Flüssigkeiten, hauptsächlich nach Erreichen der Saugkapazität, in der Regel zum Quellen und damit zu einer plastischen Konsistenz (Erweichen) sowie zum Verklumpen. Noch ausgeprägter ist dies bei Kieselgur, welche stark klebrig, schmierig und sogar breiig wird. Diese nachteiligen Eigenschaften führen zwangsläufig zu Schwierigkeiten bei der Handhabung derartiger Produkte. Bimsstein hat den Nachteil der zu geringen Saugfähigkeit. Daher wird er mit Sepiolithen vermischt verwendet, was wiederum die genannten schwerwiegenden Nachteile der letzteren mit sich bringt.

Da es sich bei dieser bekannten Ab- und Adsorptionsmitteln vorzugsweise um Naturprodukte handelt, haben sie auch noch den Nachteil, daß sie in der Regel einer größeren Variationsbreite hinsichtlich ihrer chemischen und mineralischen Zusammensetzung unterliegen und sich in der Art und Menge ihrer Verunreinigungen unterscheiden. Durch Abmischen beispielsweise mit Sand wird eine Kompensation dieser bekannten Nachteile angestrebt, was jedoch zwangsläufig infolge einer Konzentrationsverbindung der wirksamen Bestandteile zu einer verminderten Saugfähigkeit der Produkte führt, ohne die genannten nachteiligen Eigenschaften effektiv aufzuheben.

Aus der DE-A-2 640 341 sind Absorptionsmittelgranulate mit Porenstruktur, insbesondere zur Verwendung als Tierstreu, bekannt, die so hergestellt werden, daß man einen Zuschlagstoff bzw. ein Bindemittel und ein Treibmittel vermischt, die porosierte Mischung in einem Autoklaven erhitzt und erhärtet, das gehärtete Produkt zerkleinert und es schließlich siebt. Als Zuschlagstoff wird dabei Quarzsand (90 μm) bevorzugt, als Bindemittel kann Kalk oder Brandkalk verwendet werden. Dem Ausgangsgemisch wird zur Herstellung der Mikroporenstruktur ein Treibmittel, welches eine anionenaktive grenzflächenaktive Substanz, wie z. B. Seifenlösung, sein kann, zugegeben, woraufhin das gesamte Gemisch verschäumt wird. Weiterhin ist aus der USA-3 144 346 die Erzeugung von Poren in Calciumsilikatprodukten durch Mischung einer Aufschlämmung von Calciumoxid und Siliziumdioxid enthaltenden Materialien mit einem vorher erzeugten Schaum bekannt.

Der Erfindung liegt die Aufgabe zugrunde, unter Behebung der Nachteile des Standes der Technik die Verwendung von neuen Produkten mit niedrigem Schüttgewicht als Ab- bzw. Adsorptionsmittel für Flüssigkeiten, Dämpfe und Gase mit besserer Saugfähigkeit, und zwar beschleunigtem Aufsaugvermögen bei besserer Saugkapazität (Speichervolumen) und gleichmäßigem Körnungsaufbau, welche nicht quellend, nicht klebend und nicht schmierend sind, mit Harnsäure keine Geruchsbildung ergeben, sondern im Gegenteil geruchbindend und ferner bakterizid sowie nicht staubend und nicht färbend sind, zu schaffen.

Erfindungsgemäß wird diese Aufgabe durch die Verwendung von Calciumsilikatgranulaten bzw. -pulver mit Mikroporenstruktur, welches durch Umsetzen von kristallinem und gegebenenfalls amorphem Siliciumdioxid bzw. dieses oder diese enthaltenden Materialien und Calciumoxid bzw. dieses enthaltenden Materialien in einem Molverhältnis von CaO zu SiO$_2$ von 0,8 : 1 bis 1,1 : 1 unter Homogenisieren derselben in Wasser und Formen, Autoklavenhärten, Zerkleinern, Trocknen und Klassieren mit der weiteren Maßgabe, daß das Homogenisieren bei der Umsetzung durch Dispergieren der festen Ausgangsstoffe im Wasser unter Zugabe einer vorher in Wasser in einen mikroporigen stabilen Schaum überführten anionenaktiven grenzflächenaktiven Substanz durchgeführt worden ist, erhalten worden ist, als hydrophiles Ab- bzw. Adsorptionsmittel für Flüssigkeiten, Dämpfe und Gase, wobei man als Calciumsilikatgranulat bzw. -pulver ein solches mit einem Gehalt an mindestens einem primären und/oder sekundären Fettamin als Zusatz einsetzt.

Gegenstand der nicht vorveröffentlichten DE-A-2 832 194 ist ein Verfahren zur Herstellung von Calciumsilikatgranulaten bzw. -pulvern mit Mikroporenstruktur durch Umsetzen von kristallinem und gegebenenfalls amorphem Siliciumdioxid bzw. dieses oder diese enthaltenden Materialien mit Calciumoxid bzw. dieses enthaltenden Materialien in einem Molverhältnis von CaO zu SiO$_2$ von 0,8 : 1 bis 1,1 : 1 unter Homogenisieren derselben in Wasser und Formen, Autoklavenhärten, Zerkleinern, Trocknen und Klassieren, bei welchem das Homogenisieren bei der Umsetzung durch Dispergieren der festen Ausgangsstoffe im Wasser unter Zugabe einer vorher in Wasser in einen mikroporigen stabilen Schaum überführten anionenaktiven grenzflächenaktiven Substanz durchgeführt wird.

Es wurde nun überraschenderweise festgestellt, daß die so erhaltenen Produkte mit überlegenem Ergebnis als Ab- bzw. Adsorptionsmittel für Flüssigkeiten, Dämpfe und Gase verwendet werden können, wobei in der angegebenen Weise Calciumsilikatgranulate mit einem Gehalt an mindestens einem primären und/oder sekundären Fettamin als Zusatz eingesetzt werden.

Als anionenaktive grenzflächenaktive Substanz kann vorteilhaft eine solche (als Lösung) mit einem Aktivsubstanzgehalt (vor dem Lösen in Wasser) von 30 bis 60 %, beispielsweise 50 %, verwendet worden sein. Es können aber auch durch Wasserentzug durch Trocknung erhaltene pulverförmige Produkte mit einem Aktivsubstanzgehalt bis zu 100 % eingesetzt worden sein. Zweckmäßig ist die Konzentration der anionenaktiven grenzflächenaktiven Substanz in Wasser für den zum Homogenisieren zuzusetzenden mikroporigen stabilen Schaum bei Verwendung einer anionenaktiven grenzflächenaktiven Substanz mit einem Aktivsubstanzgehalt von 100 % zu etwa 1,0 bis 2,5 %, insbesondere 1,5 %, und bei Verwendung einer anionenaktiven grenzflächenaktiven Substanz mit einem Aktivsubstanzgehalt von 30 bis 60 % zu etwa 1,5 bis 3,5 %, insbesondere 2 %, gewählt worden. Soweit keine anionenaktive grenzflächenaktive Substanz mit einem Aktivsubstanzgehalt von 100 % verwendet worden ist, beziehen sich diese Konzentrationsangaben auf das Produkt, wie es tatsächlich eingesetzt wurde, das heißt das, gegebenenfalls unreine, Chemieprodukt. Beispielsweise entspricht dann einer Konzentration der verwendeten anionenaktiven grenzflächenaktiven Substanz in Wasser von 2 % bei einem Aktivsubstanzgehalt der ersteren von 50 % eine Konzentration von 1 % reiner grenzflächenaktiven Substanz in Wasser. Ein Beispiel für eine verwendbare anionenaktive grenzflächenaktive Substanz ist Natriumlaurylsulfat. Ferner ist es zweckmäßig, die Erzeugung des mikroproigen stabilen Schaumes mittels komprimierter Luft durchgeführt zu haben. Dabei ist vorteilhaft in der komprimierten Luft, die beispielsweise in einem Aktivschaumgenerator erzeugt worden ist, in feinster Verteilung die durch Verdünnen mit Wasser bereitete Lösung der anionenaktiven grenzflächenaktiven Substanz zu einem mikroporigen stabilen Schaum umgewandelt worden. Dieser Schaum hat die Konsistenz einer Schlagsahne und hat den Aktivschaumgenerator über eine Schlauchverbindung bzw. Rohrleitung verlassen und zum Dispergierarbeitsgang geführt worden sein können. Vorzugsweise ist als mikroporiger stabiler Schaum ein solcher mit einem Litergewicht von 30 bis 80 g/l, insbesondere 50 bis 60 g/l, verwendet worden. Die Menge der anionenaktiven grenzflächenaktiven Substanz richtete sich nach dem erzielbaren Schaumvolumen beim Schaumerzeugen, wie im Aktivschaumgenerator, und war in der Ausgangsmischung durch die angestrebte Rohdichte (Schüttgewicht) des herzustellenden Granulates bzw. Pulvers bestimmt. Sie war also eine Funktion

des Blähfaktors = Volumen des Fertigproduktes/Volumen der eingesetzten Feststoffmenge und auch im Hinblick auf die Stabilität des Schaumes und die Gewährleistung einer schonenden Einarbeitung desselben in die Ausgangsmischung zu wählen. Vorzugsweise ist die anionenaktive grenzflächenaktive Substanz (unverdünnt gerechnet) in einer Menge von etwa 300 bis 600 $g/m^3$ Fertigprodukt verwendet worden.

Die mikroporige Produktstruktur der erfindungsgemäß verwendeten Calciumsilikatgranulate bzw. -pulver ist von großer Bedeutung, da sie die angestrebten Produkteigenschaften, wie die hohe Saugkapazität sowie hohe Druckfestigkeit und niedrige Rohdichte (Schüttgewicht), sicherstellt.

Zweckmäßig sind als Siliciumdioxid und Calciumoxid bzw. diese enthaltende Materialien solche Rohstoffe, welche möglichst wenig Verunreinigungen enthalten, verwendet worden. Es ist bevorzugt, als kristallines Siliciumdioxid Quarzmehl, das heißt ein durch Feinmahlen von Quarzsand hergestelltes Pulver mit einer Feinheit von weniger als 100 μm verwendet zu haben. Die Mitverwendung von amorphem Siliciumdioxid bzw. dieses enthaltenden Materialien ist möglich. Vorteilhafter ist es jedoch, nur kristallines Siliciumdioxid bzw. dieses enthaltende Materialien verwendet zu haben. Vorzugsweise ist bzw. sind als Calciumoxid bzw. dieses enthaltendes Material gebrannter Kalk und/oder Kalkhydrat verwendet worden. Es ist bevorzugt, als Calciumoxid bzw. dieses enthaltende Materialien Gemische von gebranntem Kalk und Kalkhydrat verwendet zu haben. Dabei ist es besonders bevorzugt, vom Calciumoxid bzw. dieses enthaltenden Material einen Anteil von etwa 2/3 gebranntem Kalk und einen Anteil von etwa 1/3 Kalkhydrat verwendet zu haben. Dadurch ist nämlich die Löschwärme des gebrannten Kalkes zu einem bestimmten Teil vorweggenommen worden. Es kann aber auch allein gebrannter Kalk oder Kalkhydrat verwendet worden sein. Als gebrannter Kalk ist vorteilhaft Weißfeinkalk verwendet worden. Zweckmäßig ist die Zusammensetzung der festen Ausgangsstoffmischung wie folgt : Etwa 50 bis 57 Gew.-% Quarzmehl, etwa 28 bis 33 Gew.-% gebrannter Kalk und etwa 14 bis 17 Gew.-% Kalkhydrat. Wie bereits erwähnt, kann der gebrannte Kalk ganz oder teilweise durch Kalkhydrat bzw. das Kalkhydrat ganz oder teilweise durch gebrannten Kalk ersetzt gewesen sein. Vorteilhaft ist das Wasser (Summe des zur Schaumbildung erforderlichen Wassers und des Mischwassers) in einer Menge von 45 bis 70 Gew.-%, insbesondere 48 bis 60 Gew.-%, bezogen auf die eingesetzte Feststoffgemischmenge als Trockengewicht berechnet, verwendet worden. Von diesem Wasser sind zweckmäßig 8 bis 12 % als zur Schaumbildung erforderliches Wasser und 88 % bis 92 % als Mischwasser eingesetzt worden.

Zweckmäßig ist nach dem Vermischen und Bereitstellen der Ausgangsstoffe für das Formen und vor dem Autoklavenhärten eine Vorreaktion mit einer Zeitdauer von mindestens 30 Minuten durchgeführt worden. Es ist dies anders ausgedrückt die Zeit, welche nach dem Herstellen und Einfüllen der Ausgangsmischung in die Form bis zur Durchführung der Autoklavenhärtung erforderlich war. Innerhalb dieser Vorreaktionszeit ist eine ausreichende Versteifung für die Beanspruchung durch die Autoklavenhärtung mittels hochgespannten Dampfes erfolgt. Eine Verlängerung dieser Mindestvorreaktionszeit war nicht nachteilig. Vorzugsweise ist die Vorreaktion bei einer Temperatur von 70 bis 80 °C durchgeführt worden. Im Falle der Verwendung von gebranntem Kalk, wie Weißfeinkalk, ergab sich diese Temperatur zwangsläufig bei Einsatz von verhältnismäßig geringen Wassermengen durch die Reaktionswärme des gebrannten Kalkes, wobei eine Wärmezuführung anderer Art nicht zu erfolgen hatte. Im Falle der alleinigen Verwendung von Kalkhydrat war dagegen zur Erreichung der genannten Temperatur ein Vorwärmen des zugesetzten Wassers (Mischwasser) auf 70 bis 80 °C erforderlich.

Im allgemeinen sind die Porenradien der erfindungsgemäß verwendeten Calciumsilikatgranulate bzw. -pulver weniger als 100 μm vorzugsweise weniger als 60 μm, insbesondere 10 bis 20 μm.

So haben die erfindungsgemäß verwendeten Calciumsilikatgranulate bzw. -pulver eine hohe Korneigenfestigkeit und damit hohe Druckfestigkeit und ein niedriges Schüttgewicht.

Die erfindungsgemäß verwendeten Calciumsilikatgranulate bzw. -pulver können die folgenden Materialfestigkeits- und Materialdichteparameterwerte haben (hier und im folgenden bedeutet die internationale Dimension $N/mm^2$ Newton/Quadratmillimeter = 10 $kp/cm^2$) :

| | |
|---|---|
| Druckfestigkeit in $N/mm^2$ | 6,5 bis 10,2 |
| Rohdichte in $kg/m^3$ | 400 bis 700 |
| Schüttgewicht in $kg/m^3$ | 250 bis 500 |
| (Schüttgewicht in $kg/m^3$ der Korngrößen bis 2,5 mm | 300 bis 500 |
| Schüttgewicht in $kg/m^3$ der Korngrößen von 2,5 bis 4 mm | 250 bis 450). |

Ferner zeichnen sich die erfindungsgemäß verwendeten Calciumsilikatgranulate bzw. -pulver durch ein überlegen hohes und stets gleichmäßig reproduzierbares Ab- bzw. Adsorptionsvermögen aus. Ihre Saugkapazität in bezug auf Wasser (Sättigungswasseraufnahme) kann 200 Gew.-% (bei einem Schüttgewicht von 450 g/l) und mehr betragen. Noch dazu zeigen sie eine beschleunigte Aufsaugung, den sogenannten « Löschblatteffekt ».

Ein großer Vorteil der erfindungsgemäßen Verwendung ist es, daß die erfindungsgemäß verwendeten Calciumsilikatgranulate bzw. -pulver bei hoher Saugfähigkeit im Gegensatz zu den saugfähigsten Produkten des Standes der Technik selbst bei optimaler Nutzung nicht quellend, nicht klebend und nicht schmierend sind, also keinem Erweichen unterliegen, andererseits aber auch nicht stauben. Darüber hinaus sind sie auch nicht färbend.

Hinzukommt als weiterer Vorteil der gleichmäßige Körnungsaufbau der erfindungsgemäß verwendeten Calciumsilikatgranulate bzw. -pulver. Die gewünschte Korngröße kann durch Klassieren (wie mittels Siebens) eingestellt worden sein. Zweckmäßig ist die Korngröße der erfindungsgemäß verwendeten Calciumsilikatgranulate bzw. -pulver bis 5 mm, wobei sich zur angestrebten hydrophilen Ab- bzw. Adsorption Korngrößen von 1 bis 4 mm besonders bewährt haben.

Ein weiterer Vorteil der erfindungsgemäßen Verwendung ist es, daß bei erfindungsgemäß verwendeten Calciumsilikatgranulaten bzw. -pulvern auch bei optimaler Flüssigkeitssättigung unter Druckbelastung kein Flüssigkeitsaustritt stattfindet.

Eine zweckmäßige Ausführungsform der erfindungsgemäßen Verwendung besteht in der zur hydrophilen Ab- bzw. Adsorption. Unter « hydrophiler Ab- bzw. Adsorption » ist die Ab- bzw. Adsorption von Wasser und wäßrigen Medien, beispielsweise Urin unter Einschluß des Vorliegens derselben in Dampfform zu verstehen.

Die erfindungsgemäß verwendeten Calciumsilikatgranulate bzw. -pulver haben einen alkalischen pH-Wert von im allgemeinen 9 bis 10, vorzugsweise 9,4 bis 9,8. Daher wirken sie säurebindend. Beispielsweise vermögen sie die geruchbildenden Verbindungen, wie Harnsäure, im Tierharn chemisch zu neutralisieren und damit ohne Zusätze geruchbindend zu wirken, was einen großen Vorteil der erfindungsgemäßen Verwendung darstellt und einen weiten Bereich derselben unter Erstreckung auch auf Gebiete mit ganz speziellen Bedingungen ermöglicht.

Ein weiterer Vorteil der erfindungsgemäßen Verwendung ist die bakterizide Wirkung der erfindungsgemäß verwendeten Calciumsilikatgranulate bzw. -pulver.

Aufgrund dieser Tatsache besteht eine besonders bevorzugte spezielle Ausführungsform der erfindungsgemäßen Verwendung in der als Tierstreu. Ganz besonders bevorzugt ist die Verwendung als Katzenstreu. Gegenüber der Verwendung der bekannten Produkte dieses Gebietes bringt die erfindungsgemäße Verwendung den großen Vorteil mit sich, daß bei überlegenem Ab- bzw. Adsorptionsvermögen (hohe Saugkapazität und beschleunigte Aufsaugung) der erfindungsgemäß verwendeten Calciumsilikatgranulate bzw. -pulver diese auch noch wie bereits erwähnt nicht nur form- und konsistenzbeständig, sondern auch geruchbindend und bakterizid sind.

Eine weitere spezielle Ausführungsform der erfindungsgemäßen Verwendung ist die zur Ab- bzw. Adsorption von Dämpfen aus Gasen. Ein Beispiel hierfür ist die Ab- bzw. Adsorption von Küchendunst. Bei diesem speziellen Gebiet ist die leichte Austauschbarkeit und der hygienische Charakter der erfindungsgemäß verwendeten Calciumsilikatgranulate bzw. -pulver von besonderem Vorteil. Ein weiteres Beispiel ist die Gastrocknung.

Wegen des erwähnten alkalischen pH-Bereiches der erfindungsgemäß verwendeten Calciumsilikatgranulate bzw. -pulver ist eine weitere spezielle Ausführungsform der erfindungsgemäßen Verwendung, welche im Abtrennen von sauren Bestandteilen, wie Chlorwasserstoff oder Kohlendioxid, aus Gasgemischen besteht, möglich.

Bei der erfindungsgemäßen Verwendung ist auch eine oleophile Ab- bzw. Adsorption neben einer hydrophilen Ab- bzw. Adsorption bis zur Erreichung der Saugkapazität durch Wahl des Calciumsilikatgranulates bzw. -pulvers möglich. Dies kann in der Weise von praktischer Bedeutung sein, daß das Öl ab- bzw. adsorbiert aufweisende Calciumsilikatgranulate bzw. -pulver durch Wasserabsorption bzw. -adsorption zum Absinken gebracht wird.

Ferner kann der Charakter der erfindungsgemäß verwendeten Calciumsilikatgranulate bzw. -pulver durch Zusätze und deren Menge in Richtung auf mehr hydrophiles Ab- bzw. Adsorptionsverhalten zu oder in Richtung auf mehr oleophiles Ab- bzw. Adsorptionsverhalten zu gesteuert, also zu Gunsten der Ab- bzw. Adsorption der einen oder anderen Flüssigkeit, die sich in ihrer Oberflächenspannung unterscheiden, geändert werden.

Besonders bevorzugt ist es, als Calciumsilikatgranulat bzw. -pulver ein solches, welches als Fettamin(e) ein solches bzw. solche, dessen bzw. deren Alkylrest(e) 16 bis 20 bzw. je 16 bis 20, insbesondere 18 bzw. je 18, Kohlenstoffatome aufweist bzw. aufweisen, einzusetzen. Bei dieser Ausführungsform ist die hydrophile Ab- bzw. Adsorption bei oleophobem Charakter begünstigt.

Es ist zweckmäßig, als Calciumsilikatgranulat bzw. -pulver ein solches, in welches das Fettamin bzw. die Fettamine durch Zugabe zur Herstellung der Ausgangsmischung vor deren Umsetzen eingebaut worden ist bzw. sind, einzusetzen. Dabei müssen natürlich die Mengen, wie die oben angegebenen zweckmäßigen Mengenbereiche, der übrigen Ausgangsstoffe, entsprechend anteilig vermindert sein. Überraschenderweise haben nämlich die genannten Zusatzstoffe die wesentliche Eigenschaft, daß sie das Verfahren zur Herstellung der erfindungsgemäß verwendeten Calciumsilikatgranulate bzw. -pulver und deren gewünschte Produktcharakteristik vermöge ihrer chemischen Beständigkeit gegenüber den verfahrensbedingten chemischphysikalischen Einwirkungen nicht beeinträchtigen. So können die erforderlichen Herstellungsverfahrensschritte, beispielsweise die Temperaturbehandlung bei etwa 200 °C unter den Bedingungen der Hydrothermalhärtung, ohne Beeinträchtigung uneingeschränkt durchgeführt worden sein, und zwar ohne daß die angestrebten Produkteigenschaften in ihrer Wirksamkeit hinsichtlich der erzielten überlegenen Saugfähigkeit in Ausrichtung auf Flüssigkeiten mit verschiedener Oberflächenspannung abgebaut und die sonstigen Produktparameterwerte beeinträchtigt würden.

Im Falle eines Fettamins bzw. von Fettaminen als Zusatz ist es bevorzugt, daß dessen bzw. deren Zugabe zur Herstellungsausgangsmischung in einer Menge von 0,05 bis 0,5 Gew.-%, insbesondere 0,05 bis 0,3 Gew.-%, ganz besonders 0,25 Gew.-%, bezogen auf die eingesetzte Feststoffgemischmenge als Trockengewicht berechnet, erfolgt ist. Dabei sind diese Zusätze vorteilhaft in Form von, vorzugsweise 5 bis 15 %igen, insbesondere 10 %igen, wäßrigen Dispersionen zugegeben worden. Die Menge der Dispersion muß natürlich der zuzugebenden Zusatztrockenmenge angepaßt worden sein. Beispielsweise im Falle einer 10 %igen wäßrigen Dispersion ist der bevorzugte Mengenbereich derselben 0,5 bis 5 Gew.-%, insbesondere 0,5 bis 3 Gew.-%, ganz besonders 2,5 Gew.-%, bezogen auf die eingesetzte Feststoffgemischmenge als Trockengewicht berechnet.

Wie bereits erwähnt, sind die erfindungsgemäß verwendeten Calciumsilikatgranulate bzw. -pulver an sich schon geruchbindend. Es ist aber auch möglich, solche mit einem Zusatz von Deodorierungsmitteln, wie Parfümölen, die auf sie aufgebracht worden sein können, zu verwenden. Dies ist insbesondere bei der speziellen Verwendung als Tierstreu von Bedeutung.

Gegebenenfalls können erfindungsgemäß Calciumsilikatgranulate bzw. -pulver mit einem Zusatz von Farbindikatoren zur Kontrolle des Sättigungsgrades der Ab- bzw. Adsorption verwendet werden.

**Ansprüche**

1. Verwendung von Calciumsilikatgranulat bzw. -pulver mit Mikroporenstruktur, welches durch Umsetzen von kristallinem und gegebenenfalls amorphem Siliciumdioxid bzw. dieses oder diese enthaltenden Materialien und Calciumoxid bzw. dieses enthaltenden Materialien in einem Molverhältnis von CaO zu $SiO_2$ von 0,8 : 1 bis 1,1 : 1 unter Homogenisieren derselben in Wasser und Formen, Autoklavenhärten, Zerkleinern, Trocknen und Klassieren mit der weiteren Maßgabe, daß das Homogenisieren bei der Umsetzung durch Dispergieren der festen Ausgangsstoffe im Wasser unter Zugabe einer vorher in Wasser in einen mikroporigen stabilen Schaum überführten anionenaktiven grenzflächenaktiven Substanz durchgeführt worden ist, erhalten worden ist, als hydrophiles Ab- bzw. Adsorptionsmittel für Flüssigkeiten, Dämpfe und Gase, wobei man als Calciumsilikatgranulat bzw. -pulver ein solches mit einem Gehalt an mindestens einem primären und/oder sekundären Fettamin als Zusatz einsetzt.

2. Verwendung nach Anspruch 1, dadurch gekennzeichnet, daß man als Calciumsilikatgranulat bzw. -pulver ein solches, welches als Fettamin(e) ein solches bzw. solche, dessen bzw. deren Alkylrest(e) 16 bis 20 bzw. je 16 bis 20 Kohlenstoffatome aufweist bzw. aufweisen, einsetzt.

3. Verwendung nach Anspruch 1 oder 2, dadurch gekennzeichnet, daß man als Calciumsilikatgranulat bzw. -pulver ein solches, in welches das Fettamin beziehungsweise die Fettamine durch Zugabe zur Herstellungsausgangsmischung vor deren Umsetzen eingebracht worden ist beziehungsweise sind, einsetzt.

4. Verwendung nach einem der vorangehenden Ansprüche, dadurch gekennzeichnet, daß man als Calciumsilikatgranulat beziehungsweise -pulver ein solches, bei welchem die Zugabe des Fettamines beziehungsweise der Fettamine zur Herstellungsausgangsmischung in einer Menge von 0,05 bis 0,5